(19) Europäisches Patentamt
European Patent Office
Office européen des brevets

(11) **EP 0 862 907 B1**

(12) **EUROPÄISCHE PATENTSCHRIFT**

(45) Veröffentlichungstag und Bekanntmachung des Hinweises auf die Patenterteilung:
**23.04.2003 Patentblatt 2003/17**

(51) Int Cl.⁷: **A61K 7/06**, A61K 7/48,
A23J 3/14, A23J 1/14

(21) Anmeldenummer: **98100938.4**

(22) Anmeldetag: **21.01.1998**

(54) **Proteinextrakt aus Getreidekleber**

Protein extract from cereal gluten

Extrait protéinique de gluten de céréale

(84) Benannte Vertragsstaaten:
**AT BE CH DE DK ES FI FR GB GR IE IT LI LU MC NL PT SE**

(30) Priorität: **07.03.1997 DE 19709360**

(43) Veröffentlichungstag der Anmeldung:
**09.09.1998 Patentblatt 1998/37**

(73) Patentinhaber: **Dragoco Gerberding & Co Aktiengesellschaft**
**37603 Holzminden (DE)**

(72) Erfinder:
• **Manzo, Robert**
**Goshen, NY 10924 (US)**
• **Pickenhagen, Wilhelm, Dr.**
**37671 Höxter (DE)**
• **Vollhardt, Jürgen, Dr.**
**37639 Bevern (DE)**

(74) Vertreter: **Eisenführ, Speiser & Partner**
**Martinistrasse 24**
**28195 Bremen (DE)**

(56) Entgegenhaltungen:
WO-A-91/06227          US-A- 4 174 314

• DATABASE WPI [Online] DERWENT PUBLICATIONS LTD., LONDON, GB access number 1984-266717, XP002132970 & JP 59 163304 A (KASHIWA KAGAKU KOGYO K.K.) 14. September 1984 (1984-09-14)
• DATABASE CHEMICAL ABSTRACTS [Online] stn abstract 111: 102 488, XP002132971 & JP 63 253012 A (ICHIMARU CO., LTD) 20. Oktober 1988 (1988-10-20)
• DATABASE CHEMICAL ABSTRACTS [Online] stn abstract 93: 93 888, XP002132972 & JP 55 002939 A (FUJI SEIYU K.K.) 23. Januar 1980 (1980-01-23)
• DATABASE WPI [Online] DERWENT PUBLICATIONS LTD., LONDON, GB access number 1993-049522, XP002132973 & JP 05 000927 A (SHISEIDO CO., LTD) 8. Januar 1993 (1993-01-08)

**Beschreibung**

Während des Lebenszyklus eines Humanhaares werden durch mechanische Beanspruchung, z.B. beim Kämmen oder Toupieren, oder auch durch chemische Behandlung wie z.B. Bleiche, Färbung oder Dauerwelle mehr oder weniger starke Schäden an der Struktur des Haares verursacht. Diese Schäden verschlechtern die Oberflächeneigenschaften des Haares, z.B. bezüglich Glanz, Geschmeidigkeit oder Kämmbarkeit und erniedrigen allgemein die Festigkeit. Geschädigte Haare können leichter brechen als ungeschädigte.

Es ist seit längerer Zeit bekannt, daß Proteinhydrolysate, die sich durch Abbau nativer Proteine herstellen lassen, haarpflegende Eigenschaften besitzen. Diese Hydrolysate enthalten hauptsächlich Peptide mit einem Molekulargewicht im Bereich von 2 - 3 kDa und lassen sich aus verschiedenen Proteinquellen gewinnen, beispielsweise aus Getreideklebern dadurch, daß der Kleber entfettet und dann unter alkalischen Bedingungen extrahiert wird. Als Beispiel beschreibt die eigene WO-A 90/05521 ein Hydrolysat, das in einem mehrstufigen Verfahren aus Getreidekleber, vorzugsweise Weizenkleber gewonnen wird und u.a. in Haarpflegemitteln einsetzbar ist. Gemäß dieser Schrift wird der getrocknete Kleber zunächst mit einem Fettlösemittel entfettet und danach mit wässrigem Ethanol, der durch $NH_3$ alkalisch eingestellt ist, extrahiert. Der Extraktionsrückstand wird verworfen, und die flüssige Phase wird ggf. unter Vollvakuum abgekühlt, wobei ein Proteinprodukt ausfällt, das nach Abtrennung eine bei Raumtemperatur honigartige klare Hydrolysat-Masse bildet. Diese Masse kann für die gewünschten Zwecke konditioniert und weiterverarbeitet werden.

Derartige Hydrolysate werden verhältnismäßig gleichmäßig vom Haar aufgenommen und lassen sich dann nicht nur in der äußeren Schuppenzellschicht (Cuticula) des Haares, sondern auch im Bereich des Faserstammes (Cortex) finden. Ein solches Verhalten ist zur mehr vorbeugenden Pflege von noch weitgehend ungeschädigtem Haar sehr erwünscht. Für bereits stärker geschädigtes Haar ist hingegen ein Pflegereagenz anzustreben, das in der Lage ist, den Ort der Schädigung zu erkennen, sich spezifisch dort anzulagern und den Auswirkungen der Schädigung entgegen zu wirken. Es ist das Ziel der Erfindung, ein solches Pflegereagenz zur Verfügung zu stellen.

Dieses Ziel wird erfindungsgemäß erreicht mit einem Proteinextrakt, der dadurch hergestellt ist, daß der getrocknete Getreidekleber, vorzugsweise Weizenkleber, mit verdünntem kurzkettigen Alkanol, vorzugsweise Ethanol extrahiert wird, die flüssige Phase nach Abtrennung des festen Rückstands mit Glycerin oder ggf. einem kurzkettigen Alkandiol, wie beispielsweise Ethandiol oder Propandiol versetzt und solange im Vakuum eingeengt wird, bis sich der Alkanolgehalt der flüssigen Phase auf weniger als 5 Gew.%, vorzugsweise weniger als 1 Gew.% vermindert hat. Vorzugsweise wird dabei die Extraktion mit 50 - 90 %igem, bevorzugt 60 - 70 %igem Ethanol als Extraktionsmittel im Verhältnis Kleber zu Extraktionsmittel von 1 : 15 bis 1 : 2, bevorzugt 1 : 5 und bei Temperaturen von 15 - 45 °C, bevorzugt bei 20 °C durchgeführt. Die Extraktionszeit kann dabei 0,25 - 48 h betragen und liegt, abhängig von den verwendeten Geräten, normalerweise im Bereich von 15 h. Der Proteingehalt des eingeengten Extrakts liegt bei 1 - 30 % Protein, vorzugsweise bei 10 Gew.% Protein.

Die Erfindung bzw. ihre bevorzugten Ausgestaltungen unterscheiden sich von der WO-A 90/05521 sowohl verfahrensmäßig als auch im Produkt. So wird ein nicht entfetteter Kleber der Extraktion unterworfen, wodurch sich ein höherer Gehalt an Lipiden, Glycolipiden und Phospholipiden im Extrakt ergibt. Desweiteren wird die Extraktion nicht im alkalischen Bereich, sondern im Neutralbereich durchgeführt, so daß kaum Hydrolyse stattfindet und ganz anders zusammengesetzte Proteinfraktionen anfallen. Außerdem erfolgt auch keine Ausfällung der Proteine aus dem Extrakt, wodurch vermieden wird, daß gut lösliche Komponenten im Überstand der Fällung angereichert und mit dem überstand verworfen werden. Schließlich ist das Produkt gemäß der WO-A 90/05521 eine honigartige Masse, die schlecht handhabbar ist, ggf. mit Ethanol/Wasser verdünnt werden muß und dann zur Bildung von Niederschlägen neigt, wodurch die weitere Verwendbarkeit nachhaltig eingeschränkt wird. Das Produkt gemäß der Erfindung ist dagegen eine klare Lösung von ganz ausgezeichneter Lagerstabilität, die problemlos in kosmetische Präparate eingebracht werden kann. Es enthält auch keinen oder zumindest weniger als 5 % Ethanol, das in höheren Konzentrationen u.U. den Flammpunkt und die Viskosität kosmetischer Präparate unerwünscht erniedrigen kann.

Als entscheidend dürfte anzusehen sein, daß der Proteinextrakt gemäß der Erfindung kein Proteinhydrolysat darstellt, sondern im wesentlichen die nativen Getreideproteine (Prolamine) enthält (im Falle des Weizens also das Gliadin), die im Unterschied zu den Hydrolysaten kaum wasserlöslich sind und in der Hauptfraktion ein Molekulargewicht von 28 - 39 kDa aufweisen. Überraschend wurde nun festgestellt, daß dieses Produkt - anders als die Hydrolysate - das Haar nicht mit einem gleichmäßigen Film belegt und auch nicht in tiefere Haarbereiche hineindiffundiert, sondern sich primär an geschädigte Stellen des Humanhaares anbindet, und zwar besonders stark an gespaltenen Haarenden oder an durch Kämmen oder Toupieren geschädigten Haarabschnitten. Damit ist durch die Erfindung ein spezifisch für geschädigtes Haar geeignetes Pflegereagenz zur Verfügung gestellt.

Die nachfolgenden Beispiele erläutern die Erfindung

Beispiel 1 **Herstellung des Proteinextrakts**

Kommerziell erhältlicher, getrockneter Weizenkleber wird mit 50 - 90 %igem, vorzugsweise 60 - 70 %igem Ethanol im Verhältnis Weizenkleber zu Lösungsmittel von 1 : 15 bis 1 : 2, vorzugsweise 1 : 5 bei 15 - 45 °C, vorzugsweise bei 20 °C extrahiert.

Nach einer Rührzeit von 0,25 - 48 h, vorzugsweise 15 h wird die Suspension mittels eines Dekanters weitgehend von der festen Phase befreit. Das so gewonnene Dekantat wird durch Filtration mit einem Plattenfilter klar filtriert. Dann wird Glycerin zugesetzt und im Vakuum eingeengt. Wasser und Ethanol werden solange abdestilliert bis weniger als ca. 1 % Ethanol im Sumpfprodukt enthalten sind. Das so enthaltene Produkt kann 1 - 30 Gew.% Protein enthalten und enthält normalerweise etwa 10 Gew.% Protein (Trockensubstanz).

Anstelle von oder zusammen mit Ethanol können auch Methanol und/oder Isopropanol in geeigneter Verdünnung zur Extraktion eingesetzt werden und das Glycerin kann ganz oder teilweise durch ein Alkandiol wie Ethandiol und/oder Propandiol ersetzt werden.

Beispiel 2 **Nachweis spezifischer Adsorption an Humanhaar**

2.1. Herstellung des fluoreszenzmarkierten Proteins

Das nach Beispiel 1 aus Weizenkleber extrahierte Protein wurde, um es "sichtbar" zu machen, mit Fluorescein Isothiocyanat (FITC 1 on Celite, Firma Aldrich) bei Raumtemperatur umgesetzt.

Zu 1,5 g Protein wurden 37,5 mg FITC 1 gegeben und der pH-Wert der Ethanol/Wasser-Lösung mit Triethylamin p.A. auf 8,5 eingestellt. Der Reaktionsansatz wurde 2 h bei RT vor Licht geschützt gerührt. Der Rückstand (Celite) wurde durch Zentrifugieren (bei 3000 rpm, 10 min) abgetrennt, der überstand mit einer Sephadex LH-20 Säule von niedermolekularen FITC und vorhandenen Salzen gereinigt. Die fluoreszierenden Fraktionen wurden gesammelt und am Rotationsverdampfer eingeengt.

Ausbeute: 115 mg

2.2. Haarbehandlungen zur Erzeugung definierter Haarschädigung

2.2.1. Kämmen

Jeweils 2 cm breite Haartressen wurden mit einem gepreßten Kamm insgesamt 2000mal (von jeder Seite 1000mal) in Richtung von der Wurzel zur Spitze gekämmt.

2.2.2. Toupieren

Eine 2 cm breite Haartresse wurde insgesamt 50mal mit einem gepreßten Kamm in Richtung von der Spitze zur Wurzel toupiert.

2.2.3. Bleichen

Jeweils 100 mg Humanhaare, die zu einer Strähne zusammengebunden waren, wurden in einer Lösung aus 9,6 ml $H_2O$, 2,4 ml $H_2O_2$ (30 %ig), 0,57 mg Ammoniumcarbonat bei pH 9,1 eine Stunde unter schwachem Rühren bei 30 °C in einem Thermostaten behandelt. Es wurde sorgfältig mit Wasser gewaschen und an der Luft getrocknet.

2.3. Behandlung der Haare aus ethanolischer Lösung und Erzeugung der Waschflotte zur Untersuchung gemäß 2.5.

Für die Behandlungen wurden 10 ca. 5 cm lange Haare zu einer Strähne zusammengebunden. Die Behandlungen erfolgten in einer Lösung aus Ethanol/Wasser (70/30, v/v) mit 0,5 % (m/v) Proteingehalt (fluoreszenzmarkiert nach 2.1.) 30 min unter Schütteln bei RT. Zum Spülen der Haare werden 25 ml verwendet.

2.4. Behandlung aus wässriger Natriumlaurethsulfat-Lösung und Erzeugung der Waschflotte zur Untersuchung gemäß 2.5.

Für die Behandlungen wurden 10 ca. 5 cm lange Haare zu einer Strähne zusammengebunden. Die Behandlungen erfolgten in einer Lösung aus 5 %iger (m/v) SLS-Lösung mit 0,5 % (m/v) Proteingehalt (fluoreszenzmarkiert nach 2.1.) 30 min unter Schütteln bei RT. Zum Spülen der Haare werden 25 ml verwendet.

2.5. Fluoreszenzspektroskopie

Die Fluoreszenzspektroskopie der Waschflotten aus 2.4. und 2.5. erfolgte mittels eines LS50 Luminszenzspektrometers (Perkin-Elmer, Überlingen) in Spezialquarzglasküvetten (Schichtdicke: 1 cm). Alle Fluoreszenzmessungen wurden relativ zu einem Rhodamin 101-Standard (3 %ig in Ethylenglykol) gemacht. Die Anregungs- und Emissionsspalte war auf 5,0 nm begrenzt.

2.6. Ergebnis

Tab. 1

| Fluoreszenzintensitäten $I_{max}$ (in %) der Waschflotte aus 2.3. bei Verwendung gemäß 2.2. vorbehandelter Humanhaare ($\lambda_{ex}$ = 494 nm) | |
|---|---|
| **Haarmaterial** | **$I_{max}$ in % (bei 522 nm)** |
| unbehandelte Haare | 38,37 |
| gebleichte Haare | 9,69 |
| 2000mal gekämmte Haare | 9,16 |
| 50mal toupierte Haare | 34,39 |

Tab. 2.

| Fluoreszenzintensitäten $I_{max}$ (in %) der Waschflotte aus 2.4. bei Verwendung gemäß 2.2. vorbehandelter Humanhaare ($\lambda_{ex}$ = 494 nm). | |
|---|---|
| **Haarmaterial** | **$I_{max}$ in % (bei 522 nm)** |
| unbehandelte Haare | 112,87 |
| gebleichte Haare | 12,33 |
| 2000mal gekämmte Haare | 14,74 |
| 50mal toupierte Haare | 16,20 |

Das fluoreszenzmarkierte Protein wurde auf geschädigtem Haar wesentlich besser festgehalten und führte deshalb beim Auswaschen zu kleineren Fluoreszenzintensitäten.

Beispiel 3 **Fluoreszenzmikroskopische Beurteilung**

Haare wurden gemäß Beispiel 2.3. und Beispiel 2.4. (ohne Auswaschen) mit Lösungen des fluoreszenzmarkierten Proteins behandelt und anschließend getrocknet und mittels Fluoreszenzmikroskopie mittels eines Scanning Photometermikroskops MPMO3 (Zeiss, Oberkochen) in der Auflichttechnik durchgeführt. Als Lichtquelle diente eine Quecksilberhöchstdrucklampe. Für die Fluoreszenzmikroskopie wurde ein spezieller Reflektor, der eine Anregung der Fluoreszenz bei 450 - 490 nm und eine Emission oberhalb einer Wellenlänge von 520 nm ermöglicht, verwendet. Die Haare wurden für die fluoreszenzmikroskopische Untersuchung in Immersionsöl (Zeiss) eingebettet.

Für die Faserquerschnittbereitung wurden ca. 10 Haare in ein Acrylatharz (Historesin 0, Leica, Bensheim) eingebettet. Nach Aushärten des Harzes wurden mit Hilfe eines Rotationsmikrotoms Supercut 2050 (Leica) Faserquerschnitte in Schnittdicken von 20 µm bereitet.

Auf den Mikroskopiebildern erkennt man keine gleichmäßige Belegung der Haare mit Protein. Vor allem wird eine Belegung der Schuppenkanten beobachtet. An den mechanisch vorgeschädigten Haaren kann eine deutlich stärkere Belegung im Vergleich zu unbehandelten Haaren nachgewiesen werden, besonders in den Spitzenabschnitten. Auch gebleichte Haare zeigen eine deutlich stärkere Adsorption des Proteins.

Die Aufnahme von Haarquerschnitten zeigten ringförmige Fluoreszenzerscheinungen, die zeigen, daß das Protein nur im Außenbereich des Haares angelagert wird.

Beispiel 4 **Beispiel für die Wirkung eines Gliadin enthaltenden erfindungsgemäßen Proteinextrakts**

Durch die Untersuchungen mit fluoreszenzmarkiertem Protein (Gliadin) gemäß Beispiel 2 und 3 konnte eine bevorzugte Deposition an geschädigten Haarpartien gezeigt werden. Dadurch wird eine Stärkung des Haares bewirkt, die durch Zugspannungsprüfung nachgewiesen werden kann.

Haarmaterial und Vorbehandlung

Dazu wurde europäisches Haarmaterial eingesetzt, das in der Form von Strähnen von 23 cm Länge und ca. 2,4 g Gewicht erhältlich ist. Diese Strähnen wurden nach praxisnahen Standardrezepten zunächst einer Bleiche, dann im glatten Zustand einer alkalischen Dauerwelle mit Thioglykolsäure und abschließend einer Wäsche mit Laurylethersulfat mit nachfolgender 1 %-iger Zitronensäurespülung unterzogen. Anschließend wurden die Strähnen abgequetscht, unter wiederholtem Kämmen unter einer Trockenhaube getrocknet und abschließend 24 Stunden bei 65 % rel. Luftfeuchte und 20° C konditioniert.

Behandlung mit einem Shampoo auf Basis eines erfindungsgemäßen Proteinextrakts (Gliadin Shampoo)

Die Strähnen wurden dann zu Vergleichszwecken mit einem erfindungsgemäßen "Gliadin Shampoo" und einem nicht erfindungsgemäßen "Placebo Shampoo" behandelt. Dazu wurden die Strähnen zunächst 15 min genetzt und nachfolgend in einen "handtuchtrockenen" Zustand gebracht. Die Shampoos wurden im Verhältnis 1 : 4, bezogen auf das Gewicht der trockenen Strähnen, aufgebracht und einmassiert, bei einer Gesamteinwirkzeit von 3 min. Nachfolgend wurden die Strähnen 2 - 3 min unter fließendem Wasser (ca. 35° C) ausgespült und wie oben beschrieben getrocknet. Diese Behandlung wurde 20 mal wiederholt.

Shampooformulierungen

| Rohstoff (Handelsnamen) | INCI name | Gliadin Shampoo Anteil [%] | Placebo Shampoo Anteil [%] |
|---|---|---|---|
| Genapol LRO flüssig | Sodium Laureth Sulfate | 37,0 | 37,0 |
| Rewoteric AMB 14 | Cocoamidopropyl Betain | 6,0 | 6,0 |
| Comperlan 100 | Cocamide MEA | 0,5 | 0,5 |
| Citronensäure | Citric Acid | 0,3 | 0,3 |
| Trilon B Pulver | Tetrasodium EDTA | 0,1 | 0,1 |
| Natriumbenzoat | Sodium Benzoate | 0,5 | 0,5 |
| Natriumchlorid | Sodium Chloride | 1,8 | 1,8 |
| Parfümöl | Fragrance | 0,5 | 0,5 |
| erfindungsgemässe Gliadinlösung (9 %ig) | -- | 2,0 | 0,0 |
| Wasser | Aqua | 51,3 | 53,3 |

Bündelzugtest

Die Messungen zur Bündelzugfestigkeit wurden in Analogie zum Standard IWTO-32-82 in Wasser (naß) oder unter Bedingungen des Normklimas (trocken = 20° C, 65 % rel. Feuchte) durchgeführt. Dazu wurden aus Strähnen für jedes Produkt zufällig insgesamt jeweils 30 Haare entnommen und zu einer Probe vereinigt. Aus der Probe wurden jeweils 10 Haare entnommen und parallel zueinander in eine Spezialklemme eingebracht. Für insgesamt neun solcher Unterproben wurden die individuellen Bruchspannungen und -dehnungen ermittelt. Nach dem Test wurden, im Falle der Naß-Messung, die Proben getrocknet (50° C/12 Std.) und nachfolgend rückkonditioniert. Die Proben wurden gewogen, um die mittlere Querschnittsfläche der Haare in jeder Unterprobe zu bestimmen.
Die Tests wurden wie folgt durchgeführt:

*   Gerät:        INSTRON Zugprüfmaschine
*   Einspannlänge:        10 mm
*   Dehnungsgeschwindigkeit:        10 mm/min

* Vollast: 10 N - 20 N
* Medium: in Wasser (naß) oder im Normklima (trocken)
* Anzahl der Haare: 9 x 10

Meßergebnisse

|  | Mittelwerte Bruchdehnung | | Mittelwerte Bruchspannung | |
|---|---|---|---|---|
|  | naß | trocken | naß | trocken |
| Gliadin Shampoo | 60,5 % | 54 % | 155 MPa | 204 MPa |
| Placebo Shampoo | 57,4 % | 53,8 % | 138 MPa | 189 MPa |

Interpretation

Durch die erfindungsgemäße Verwendung eines Proteinextrakts, im vorliegenden Beispiel durch die Verwendung eines Proteinextrakts auf Basis von Gliadin ("Gliadinlösung"), wird somit die Festigkeit des Haares entscheidend gestärkt und einer Beschädigung, z.B. durch Abreißen beim Kämmen, entgegengewirkt.

Beispiel 5 **Messung des Haarglanzes**

Testaufbau

Haarsträhnen wurden wie in Beispiel 4 vorbehandelt und dann mit dem erfindungsgemäßen Gliadin Shampoo und dem nicht erfindungsgemäßen Placebo Shampoo aus Beispiel 4 entsprechend der dortigen Prozedur behandelt.
Aus den jeweiligen Strähnen für jedes Shampooprodukt wurden insgesamt zehn Haare zufällig entnommen und im Mittelbereich an jeweils zwei Stellen vermessen. Diese Messung erfolgte mittels eines Grünlichtlasers (532 nm) bei einem Einstrahlwinkel von 40° und simultaner Erfassung des Streulichts in einem Winkelbereich von 50 - 170°. Die Einstrahlung erfolgte in der sog. Wurzel/Spitze-Richtung.
Durch Anpassung von zwei Normalverteilungen an das Streulichtprofil wird der Anteil der gerichteten (gR) und der diffusen (dR) Reflexion bestimmt. Daraus wird die Glanzzahl Gl gemäß:

$$Gl = gR/(dR + gR) \times 100 \%$$

berechnet.

Meßergebnisse

|  | Mittelwerte Glanzzahl |
|---|---|
| Gliadin Shampoo | 48,0 % |
| Placebo Shampoo | 40,1 % |

Interpretation

Durch die erfindungsgemäße Verwendung eines Proteinextraktes, im Beispiel durch die Verwendung eines Extraktes auf Basis von Gliadin, wird der Glanz des Haares wesentlich erhöht.

**Patentansprüche**

1. Proteinextrakt, erhältlich durch ein Verfahren mit folgenden Schritten:

- Extraktion getrockneten Getreideklebers mit verdünntem Alkanol,
- Abtrennen der resultierenden flüssigen Extrakt-Phase vom festen Rückstand,
- Versetzen der abgetrennten flüssigen Extrakt-Phase mit Glycerin und/oder einem kurzkettigen Alkandiol,
- Einengen der resultierenden flüssigen Phase im Vakuum, bis sich ihr Alkanolgehalt auf weniger als 5 Gew.%, vorzugsweise weniger als 1 Gew.% vermindert hat.

2. Verfahren zur Herstellung eines Proteinextrakts, wobei getrockneter Getreidekleber, vorzugsweise Weizenkleber, mit verdünntem Alkanol extrahiert wird, die flüssige Phase nach Abtrennung des festen Rückstands mit Glycerin und/oder einem kurzkettigen Alkandiol versetzt und solange im Vakuum eingeengt wird, bis sich der Alkanolgehalt der flüssigen Phase auf weniger als 5 Gew.%, vorzugsweise weniger als 1 Gew.% vermindert hat.

3. Verfahren nach Anspruch 2, wobei die Extraktion mit 50 - 90 %igem, bevorzugt 60 - 70 %igem Ethanol als Extraktionsmittel durchgeführt wird.

4. Verfahren nach Anspruch 2 oder 3, wobei die Extraktion mit Weizenkleber durchgeführt wird, und zwar im Verhältnis Kleber zu Extraktionsmittel von 1 : 15 bis 1 : 2, bevorzugt 1 : 5.

5. Verfahren nach einem der Ansprüche 2 - 4, wobei die Extraktion bis zu 48 h lang bei Temperaturen von 15 - 45 °C, bevorzugt bei 20 °C durchgeführt wird.

6. Verwendung des Proteinextrakts nach Anspruch 1 in kosmetischen Präparaten zur Pflege von geschädigtem Haar.

**Claims**

1. Protein extract obtainable by a method having the following steps:

- extraction of dried cereal gluten with dilute alkanol,
- separation of the resulting liquid extract phase from the solid residue,
- addition of glycerol and/or a short-chain alkanediol to the liquid extract phase separated off,
- concentration of the resulting liquid phase under vacuum until its alkanol content has fallen to less than 5 % (m/m), preferably less than 1 %(m/m).

2. Method for the preparation of a protein extract wherein dried cereal gluten, preferably wheat gluten, is extracted with dilute alkanol, glycerol and/or a short-chain alkanediol is added to the liquid phase after separating off from the solid residue and the liquid phase is concentrated under vacuum until the alkanol content of the liquid phase has fallen to less than 5 %(m/m), preferably less than 1 %(m/m).

3. Method according to Claim 2, wherein the extraction is carried out with 50 - 90 %, preferably 60 - 70 % ethanol as the extractant.

4. Method according to Claim 2 or 3, wherein the extraction is carried out with wheat gluten and specifically in a gluten to extractant ratio of 1:15 to 1:2, preferably 1:5.

5. Method according to one of Claims 2 - 4, wherein the extraction is carried out for up to 48 h at temperatures of 15 - 45 °C, preferably at 20 °C.

6. Use of the protein extract according to Claim 1 in cosmetic preparations for the care of damaged hair.

**Revendications**

1. Extrait protéinique, pouvant être obtenu par le biais d'un procédé comportant les étapes suivantes :

- extraction du gluten de céréales séché avec de l'alcanol dilué,
- séparation de la phase d'extrait liquide résultante du surnageant solide,
- mélange de la phase d'extrait liquide séparée avec de la glycérine et/ou un alcanediol à chaîne courte,
- réduction de la phase liquide résultante sous vide, jusqu'à ce que la teneur en alcanol soit réduite à moins de 5 % en poids, de préférence moins de 1 % en poids.

2. Procédé de préparation d'un extrait protéinique, selon lequel le gluten de céréales séché, de préférence le gluten de blé, est extrait avec de l'alcanol dilué, la phase liquide est mélangée, après séparation du surnageant solide, avec de la glycérine et/ou un alcanediol à chaîne courte et est réduite sous vide jusqu'à ce que la teneur en alcanol de la phase liquide se soit réduite à moins de 5 % en poids, de préférence moins de 1 % en poids.

3. Procédé selon la revendication 2, selon lequel l'extraction est réalisée avec de l'éthanol à 50 - 90 %, de préférence à 60 - 70 % comme agent d'extraction.

4. Procédé selon la revendication 2 ou 3, selon lequel l'extraction est réalisée avec le gluten de blé, et ce, en un rapport gluten sur agent d'extraction allant de 1 : 15 à 1 : 2, de préférence de 1 : 5.

5. Procédé selon l'une quelconque des revendications 1 à 4, selon lequel l'extraction est réalisée pendant une durée allant jusqu'à 48 h à des températures allant de 15 à 45°C, de préférence vers 20°C.

6. Utilisation de l'extrait protéinique selon la revendication 1, dans les préparations cosmétiques destinées au soin des cheveux abîmés.